# EUROPÄISCHE PATENTSCHRIFT

(11) **EP 1 909 639 B1**
(45) Veröffentlichungstag und Bekanntmachung des Hinweises auf die Patenterteilung: **14.04.2010**
(21) Anmeldenummer: 06761776.1
(22) Anmeldetag: 07.07.2006
(51) Int. Cl.: A61B 5/00, A61B 8/00

(54) **VERFAHREN ZUR IN VIVO GEWEBEKLASSIFIZIERUNG**
METHOD FOR IN VIVO TISSUE CLASSIFICATION
PROCEDE DE CLASSIFICATION DE TISSUS IN VIVO

(30) Priorität: 19.07.2005 DE 102005034219
(43) Veröffentlichungstag der Anmeldung: 16.04.2008
(73) Patentinhaber: Nirlus Engineering AG, 23560 Lübeck (DE)
(72) Erfinder: HERRMANN, Vera, 23560 Luebeck (DE)
(74) Vertreter: von dem Borne, Andreas
(86) Internationale Anmeldenummer: PCT/DE2006/001174
(87) Internationale Veröffentlichungsnummer: WO 2007/009426

(56) Entgegenhaltungen:
- WO-A-02/15776
- WO-A-94/28795
- DE-B3- 10 311 408
- DE-C1- 4 128 744
- DE-U1- 9 422 379
- US-B1- 6 233 481

## Beschreibung

Die Erfindung betrifft ein Verfahren zur in vivo Gewebeklassifizierung, bei dem Ultraschall und Infrarotlicht in lebendes Gewebe, insbesondere den menschlichen oder tierischen Körper, eingestrahlt werden und aus dem wieder austretenden Licht auf lokale optische Parameter, insbesondere das Absorptions- und/oder Rückstreuvermögen, des Gewebes geschlossen wird, wodurch das Gewebe klassifiziert werden kann.

Ultraschalluntersuchungen zur Auffindung von abnormem Gewebe im lebenden Organismus sind seit langem Stand der Technik. Ein üblicher Anwendungsbereich ist die Mammadiagnostik, also die Erkennung von Brustkrebs bei Frauen. Malignes Gewebe, insbesondere Krebsgewebe, zeichnet sich u. a. durch andere mechanische Eigenschaften als das umgebende gesunde Gewebe aus, so dass bei der Ultraschalleinstrahlung Impedanzkontraste an Grenzflächen zur Reflexion der Schallwellen führen. Dies wird zur Lokalisierung abnormen Gewebes ausgenutzt. Die Ultraschalluntersuchung allein lässt aber noch keinen Schluss zu, ob es sich bei der aufgefundenen Gewebeanomalie um einen bösartigen Tumor handelt oder nicht. Gängig ist deshalb die Entnahme einer Probe des Tumorkandidaten (Biopsie) zur definitiven Bestimmung im Labor.

Anhand von entnommenen Proben kann das Gewebe nicht nur exakt klassifiziert werden, sondern auch seine optischen Eigenschaften lassen sich genau messen. Insbesondere zeigt sich, dass Krebszellen bestimmte Lichtwellenlängen des Nah-Infrarot(NIR)- und Mittel-Infrarot(MIR)-Spektrums wesentlich stärker absorbieren als gesunde Zellen.

Als Stand der Technik ist die von der Erfinderin stammende DE 103 11 408 B3 zu nennen.

Der menschliche Körper ist im Wellenlängenbereich zwischen etwa 600 und 1000 nm ("biologisches Fenster") wegen der Wasserbandenminima weitgehend transparent. D.h. das Licht kann tief in das Gewebe eindringen, es durchqueren oder auch zur beleuchteten Oberfläche zurückkehren. Es gibt noch weitere "transparente Fenster" im MIR-Spektrum gekennzeichnet durch geringe Wasserabsorption im Vergleich zu anderen Gewebebestandteilen, etwa zwischen 5000 und 7500 nm und sogar zwischen 10 und 25 Mikrometer.

Innerhalb solcher "transparenten Fenster" ist es möglich, für jeden einzelnen Gewebebestandteil eine Lichtwellenlänge zu spezifizieren, die von diesem Gewebeanteil gut absorbiert oder gestreut wird. Aus ex vivo Untersuchungen entnommenen Tumorgewebes ist bereits bekannt, dass manche Wellenlängen besonders charakteristisch für Krebszellen sind, u. a. weil diese bestimmte Substanzen enthalten, die im gesunden Gewebe nicht auftreten.

Die WO 94/28795 schlägt ein Verfahren vor, eine in vivo Gewebeklassifizierung durch die kombinierte Einstrahlung von fokussiertem Ultraschall und NIR-Licht vorzunehmen. Als Messsignal dient dort die aus dem Gewebe austretende transmittierte und/oder rückgestreute Strahlung im Wellenlängenbereich 600 bis 1500 nm, die sich ändert, während der Ultraschallfokus durch das Gewebe verschoben wird. Das Verschieben des Fokus ist z.B. durch geeignete Ansteuerung eines Transducer-Arrays möglich, etwa beschrieben in der US 5 322 068.

Die WO 94/28795 lehrt im Einzelnen, dass
- der Fokus kontinuierlich in drei Dimensionen durch das zu untersuchende Gewebe bewegt werden soll, um sowohl normales als auch abnormes Gewebe zu durchlaufen, damit das abnorme Gewebe anhand des "Kontrastes" zum normalen klassifiziert werden kann;
- der fokussierte Ultraschall amplitudenmoduliert einzustrahlen ist, um anhand des Einflusses der variierenden Amplitude auf das Lichtsignal das Gewebe hinsichtlich mechanischer Parameter (z.B. Relaxationszeit) zu beurteilen;
- die Fokusposition an der Stelle eines signifikanten Einflusses der Ultraschall-Amplitude auf das optische Signal festgehalten werden soll, um sodann die spektrale Zusammensetzung des eingestrahlten NIR-Lichts zu variieren;
- aus der Abhängigkeit des optischen Messsignals von der spektralen Zusammensetzung auf die Gewebepathologie zu schließen ist.

Alle genannten Maßnahmen sind sicherlich vernünftig und möglicherweise notwendig für eine umfassende biophysikalische Analyse des komplexen Zellgewebes. Bekanntlich ändern lebende Zellen z.B. ihre optischen Eigenschaften unter Druck und in Abhängigkeit von der Temperatur. Von daher sind ausführliche Variationsanalysen gewiss das Mittel der Wahl, um alle bedeutsamen Einflussgrößen auf das optische Messsignal angemessen zu berücksichtigen.

In der medizinischen Praxis ist die interessierende Fragestellung jedoch zunächst viel einfacher: soll in der Ultraschalluntersuchung erkanntes, verdächtiges Gewebe entnommen und im Labor untersucht werden, oder ist dies vielleicht vermeidbar?

Gewöhnlich ist eine Biopsie zwar für den Patienten sehr unangenehm oder sogar schmerzhaft, aber für den behandelnden Arzt doch eher mit geringem Aufwand verbunden. Die umfangreiche Messung nach der WO 94/28795 ist zur medizinischen Diagnose eher nachteilig, denn
- allein das kontinuierliche Verschieben des Ultraschallfokus (Volumen < 1 mm³) durch ein wenigstens 1000fach größeres 3D-Meßgebiet ist nur langsam möglich und somit zeitraubend;
- die Beobachtung zellmechanischer Parameter zum Auffinden maligner Areale erscheint gegenüber der gängigen Ultraschall-Reflexionsmessung recht aufwendig, auch wenn sie vielleicht ein genaueres Mapping gestattet, was aber den Mediziner nicht unbedingt interessiert (zumindest nicht bei der Früherkennung von Krebs);
- die Variation der spektralen Zusammensetzung des Messlichts erfordert durchstimmbare Lichtquellen und/oder Spektralanalysatoren, die für sich genommen bereits teure Komponenten sind, so dass die vorgeschlagene Vorrichtung erhebliche Anschaffungskosten erwarten lässt.

Neben diesen Nachteilen ist die Vorrichtung der WO 94/28795 vorrangig auf die Detektion von transmittiertem Licht ausgelegt, wenngleich eine einseitige Messvorrichtung, die nur rückgestreutes Licht misst, explizit erwähnt wird. Allerdings ist rückgestreutes Licht gemeinhin Vielfachstreuungen unterworfen, d.h. es legt einen kaum vorhersehbaren Weg von der Lichtquelle zum daneben angeordneten Detektor zurück. Damit ist es auch nicht sicher, ob das rückkehrende Licht etwa den Ultraschallfokus durchlaufen hat. Anders gesagt, besteht bei der reinen Rückstreuung das von der WO 94/28795 nicht gelöste Problem der Quellenlokalisierung für die Beiträge zum optischen Messsignal.

Die oben erwähnte DE 103 11 408 B3 beschreibt aber eine Möglichkeit, die Konzentration von Blutbestandteilen nicht-invasiv aus der Rückstreuung spezieller IR-Wellenlängen zu bestimmen, wobei ein Ultraschallfokus zur Markierung der Rückstreuregion in Innern eines Blutgefäßes positioniert wird. Das Auswerteverfahren ist darauf ausgelegt, das aus dem Fokus zurückkehrende Licht vom übrigen rückgestreuten Licht zu unterscheiden und nur für die Fokusregion optische Eigenschaften zu bestimmen. Die Vorrichtung der DE 103 11 408 B3 verwendet eine Mehrzahl von IR-Laserdioden, deren Wellenlängen von vornherein auf die Aufgabenstellung, insbesondere die Messung von Blutsauerstoff, abgestimmt sind. Für die allgemeine Gewebeuntersuchung ist die Vorrichtung nicht ohne weiteres geeignet, da sie sich u. a. auch auf die Auffindung einer geeigneten Fokusposition nach dem Doppler-Prinzip verlässt, wobei sie die Anwesenheit eines ausreichenden Volumens gerichtet fließenden Blutes voraussetzt.

Es ist nun die Aufgabe der Erfindung, den Stand der Technik dahingehend fortzubilden, dass ein vereinfachtes Verfahren zur nicht-invasiven in vivo Gewebeklassifizierung entsteht.

Die Aufgabe wird gelöst durch ein Verfahren mit den Merkmalen des Anspruchs 1. Die Unteransprüche geben vorteilhafte Ausgestaltungen an.

Eine entsprechende Vorrichtung umfasst eine Ultraschalleinrichtung, die als ein Transducer-Array mit elektronischer Kontrollvorrichtung ausgebildet ist, und Ultraschall aussenden und empfangen kann. Je nach Ansteuerung kann die Quelle wahlweise Ultraschall mit im Wesentlichen ebenen, konkaven oder konvexen Wellenfronten aussenden, d.h. sie kann insbesondere gefächert oder fokussiert in das zu untersuchende Gewebe einstrahlen. Die Fokusposition ist dabei wählbar und kann von der Kontrollvorrichtung nach externen Vorgaben im Laufe der Messung verändert werden. Die Kontrollvorrichtung kann überdies aus der Laufzeitmessung von im Gewebe reflektierten Schallwellen auf ein räumliches Zielgebiet schließen, das eine Gewebeanomalie enthält.

Die erfindungsgemäße Vorrichtung umfasst ferner wenigstens eine, bevorzugt aber eine Mehrzahl von Lichtquellen mit enger spektraler Verteilung, besonders bevorzugt sind Laserdioden. Die Anzahl der Lichtquellen und die Auswahl der jeweiligen Hauptemissionswellenlänge sollen variabel gestaltet sein, weshalb sich ein modularer Aufbau empfiehlt. Alternativ und gewiss auch in Abhängigkeit von der zukünftigen Preisentwicklung solcher Lichtquellen kann aber eine größere Anzahl (z.B. 10-20 verschiedene Wellenlängen) gleichzeitig an der Vorrichtung angeordnet sein, die dann natürlich einzeln schaltbar sein müssen.

Dabei sind grundsätzlich alle Wellenlängen aus dem NIR- und MIR-Spektralbereich von Interesse, also konkret nicht-ionisierende Strahlung mit einer Wellenlänge von wenigstens 500 nm. Natürlich wird man bei der Auswahl von Wellenlängen für die in vivo Messung nicht ohne weiteres beliebige Mikrowellenstrahlen in Betracht ziehen können oder wollen, insbesondere auch nicht für jede interessierende Wellenlänge einen Laser zur Verfügung haben. Das Hauptaugenmerk soll hier auf das "biologische Fenster" (500-1000 nm) gerichtet sein, doch die Erfindung will sich darauf nicht eingeschränkt verstanden wissen. Es kann durchaus zweckdienlich sein, bestimmte Gewebearten anhand von Wellenlängen weit außerhalb des biologischen Fensters zu klassifizieren.

Weiterhin umfasst die erfindungsgemäße Vorrichtung einen Lichtdetektor, besonders vorteilhaft ist hier ein flächiges, lichtsensitives Sensorarray (z.B. CCD-Kamera), der die rückgestreute Lichtintensität misst. Der Lichtdetektor wird von einem elektronischen Prozessrechner regelmäßig ausgelesen. Der Prozessrechner zieht dabei auch die von der Ultraschall-Kontrollvorrichtung bereitgestellten Parameter des Ultraschallfeldes heran, insbesondere Schallfrequenz, Pulsenergie und Repetitionsrate. Mit Hilfe des bereits in der DE 103 11 408 B3 beschriebenen Algorithmus wird der Beitrag des in der Region des Ultraschallfokus rückgestreuten Lichts zur Gesamtintensität isoliert.

Unter Berücksichtigung der bekannten Tiefe des Fokus unter der Gewebeoberfläche lassen sich im gesunden Gewebe typische Streuverluste des isolierten Lichtanteils im Rechner kompensieren. Nach der Kompensation wird ein Wert z.B. für den Absorptionskoeffizienten und/oder für das Rückstreuvermögen des Gewebes im Innern des Ultraschallfokus errechnet, der sich auf einzelne oder auch mehrere Wellenlängen zugleich beziehen kann.

Für die Gewebeklassifizierung ist es erforderlich, die Fokuslage an einer möglichst aussagekräftigen Position in einer erkennbaren Gewebeanomalie einzurichten. Diese fällt nicht unbedingt mit dem Schwerpunkt der durch eine Ultraschallabtastung lokalisierten Region mit veränderter akustischer Impedanz zusammen. Vielmehr ist die Anomalie gerade in Anwesenheit krankhaft veränderter Zellen besonders durch abnorme Zellchemie gekennzeichnet und somit vor allem an den optischen Parametern auszumachen.

Erfindungsgemäß soll daher die Fokuslage vollautomatisch auf der Basis der jeweils gemessenen Absorption und/oder Rückstreuung des Gewebes im Fokus verändert werden. Die Fokuslage muss dabei nicht kontinuierlich verschoben, sondern kann sprunghaft gewechselt werden. Aus dem Vergleich der Absorptions- und/oder Streukoeffizienten bei einer bestimmten Fokuslage mit dem einer oder mehrerer Vorgängerpositionen, wird algorithmisch auf eine Nachfolgerposition geschlossen, die dann beim nächsten Messvorgang von der Ultraschall-Kontrollvorrichtung eingestellt wird.

Hinter der algorithmischen Auswahl einer Sequenz von Fokuspositionen steckt nichts anderes als ein einfaches Optimlerungsproblem. Gesucht wird der Ort des Optimums für eine aus messbarer Absorption und/oder Streuung abzuleitende Kenngröße einer oder mehrerer Lichtwellenlängen innerhalb der zuvor mittels Ultraschall aufgefundenen Gewebeanomalie. Welche Kenngröße betrachtet wird oder welches Optimum man sucht, hängt von der konkreten Messaufgabe ab.

Die Erfindung sieht vor, dass man als Kenngröße die Abweichung der Absorptions- oder Streukoeffizienten im Fokus von denen im gesunden Gewebe (eine Referenz, die zu Beginn der Messung aufgenommen wird) bestimmt, und hierfür nach einem lokalen Maximum sucht.

Man wird seine Aufmerksamkeit vor allem auf die Absorption richten, wenn man beispielsweise dem Patienten zuvor einen Farbstoff verabreicht hat, der sich hauptsächlich in malignem Gewebe anreichert. In einem solchen Fall strahlt man vorteilhaft Lichtwellenlängen ein, die der Farbstoff gut absorbiert. Bei Verwendung eines derart selektiven Farbstoffs kann auf das Aufnehmen einer Referenz für gesundes Gewebe sogar verzichtet werden. Für andere Fragestellungen, z.B. die Untersuchung von Fettgewebe, ist die Betrachtung der Rückstreuung aussagekräftiger.

Die Auswahl der zu betrachtenden Kenngröße ist bei jeder Problemstellung relativ nahe liegend und der Nutzer wird sich darüber im Klaren sein, dass das aufzusuchende Optimum (i. F. Maximum) auch an irgendeiner Position im Gewebe existiert. Man kann überdies von der Stetigkeit der zu maximierenden Funktion ausgehen und wird auch rechtfertigen können, die Differenzierbarkeit der Funktion zu vermuten, so dass etwa ein Gradientenabstieg oder irgendein an anderer bekannter Optimierungsalgorithmus zur Berechnung der Sequenz der Fokuslagen (Stützstellen der Funktion) herangezogen werden kann.

Auf den genauen Algorithmus, mit dem die Optimierung berechnet wird, kommt es hier nicht an. Wichtig ist vielmehr die Erkenntnis, dass die Verschiebung des Ultraschallfokus auf der Grundlage jenes Anteils der zurück gestreuten Lichtintensität erfolgt, der zuvor dem Gewebe der Fokusregion allein zugeordnet worden ist. Der Fokus wird solange automatisch verschoben bis er an einer optimal aussagekräftigen Position im Gewebe zur Ruhe kommt.

Ist diese Position mit dem Ultraschallfokus erst einmal erfasst, empfiehlt es sich, die Absorptionskoeffizienten (und/oder Rückstreukoeffizienten) für alle verfügbaren IR-Wellenlängen einzeln zu ermitteln. Der Prozessrechner soll zusätzlich über eine Datentabelle verfügen, mit der er die Messergebnisse vergleicht. Die Tabelle enthält eine möglichst große Zahl von Gewebearten mit den jeweils bekannten optischen Parametern, wie sie etwa im Labor gemessen wurden. Dadurch kann dem Nutzer der Messvorrichtung eine Gewebeklassifizierung unmittelbar ausgegeben werden. Man muss sich allerdings dabei bewusst sein, dass die nach dem heutigen Stand verfügbaren Datentabellen auf pathologischen Befunden beruhen, d.h. dass extrahierte Gewebeproben vermessen wurden, die sich hinsichtlich Temperatur, Druck, pH-Wert oder Blutbestandteilen in der Umgebung von der in vivo Situation sehr unterscheiden können. Dies beeinflusst die optischen Parameter zum Teil erheblich.

Dennoch ist davon auszugehen, dass die Zellchemie hiervon weitgehend unberührt bleibt, so dass eine vernünftige Klassifizierung im Rahmen gewisser Toleranzen möglich ist. Das Ausmaß solcher Toleranzen zu ermitteln ist Gegenstand zukünftiger, nicht zuletzt empirischer Arbeit. Es ist aber schon jetzt klar, dass eine Abweichung der erfindungsgemäß gewonnenen optischen Parameter von den an pathologischen Proben bestimmten praktisch unvermeidlich ist und somit nur eine Wahrscheinlichkeitsaussage über die Klassifizierung des Gewebes zu erzielen ist.

Diese Wahrscheinlichkeit konkret zu berechnen und ebenfalls dem Nutzer auszugeben ist eine ganz besonders bevorzugte Ausgestaltung der Erfindung.

Im Unterschied zur DE 103 11 408 B3, daß zwar eine Klassifizierung von lebendem Gewebe erfolgt, die auf einer Kombination von Infrarotlichtanalyse und fokussiertem Ultraschall beruht, beschreibt, wird also nun die Positionierung des Ultraschallfokus Abhängigkeit vom Befund der optischen Messung erfolgen.

Beispielsweise kann auch ein Tupel von Messwerten (A1, A2, R3, A4, ...) ein solcher optischer Parameter sein, wobei etwa A1 den Absorptionskoeffizienten für die Wellenlänge 1 und R3 den Rückstreukoeffizienten für die Wellenlänge 3 bezeichnen soll. Wesentlich ist nun, dass die optischen Parameter für eine feste Fokusposition zunächst gemessen werden. Darauf schlägt der Prozessrechner zur Optimierung der Messung eine bessere Fokusposition vor, die über das US-Wandlerarray angesteuert wird. Die tatsächlichen optischen Messwerte der zweiten Fokusposition werden erfasst und gehen in eine neue Schätzung des Prozessrechners ein usw.

So wird iterativ und automatisch eine maximal aussagekräftige Fokusposition gefunden (ohne das allmähliche Verschieben durch das Gewebe, was sehr zeitaufwendig wäre), an der die Klassifikation vorgenommen wird.

Die Gewebeklassifizierung nach optimaler Positionierung des Ultraschall-Fokus gemäß dem in der Anmeldung beschriebenen Verfahren setzt voraus, dass das am Lichtdetektor erfasste optische Signal einen unmittelbaren Rückschluss auf optische Gewebeparameter an der aktuellen Fokuslage zulässt.

Insbesondere für rückgestreutes Licht ist die präzise Quellenlokalisierung aufgrund der Vielfachstreuung von Photonen im lebenden Gewebe nicht-trivial. Das optische Messsignal wird dort zwar auch in der genannten Schrift zur Stoffanalyse benutzt, jedoch verlässt sich die Fokus-Positionierung auf die Nutzung des akustischen Doppler-Effektes in Anwesenheit ausreichend stark strömenden Blutes. Eine Anwendung in beliebigem Gewebe abseits der großen Blutgefäße ist dort jedoch nicht beschrieben.

Die Erfindung wird im Folgenden noch näher erläutert anhand der einzigen Figur:

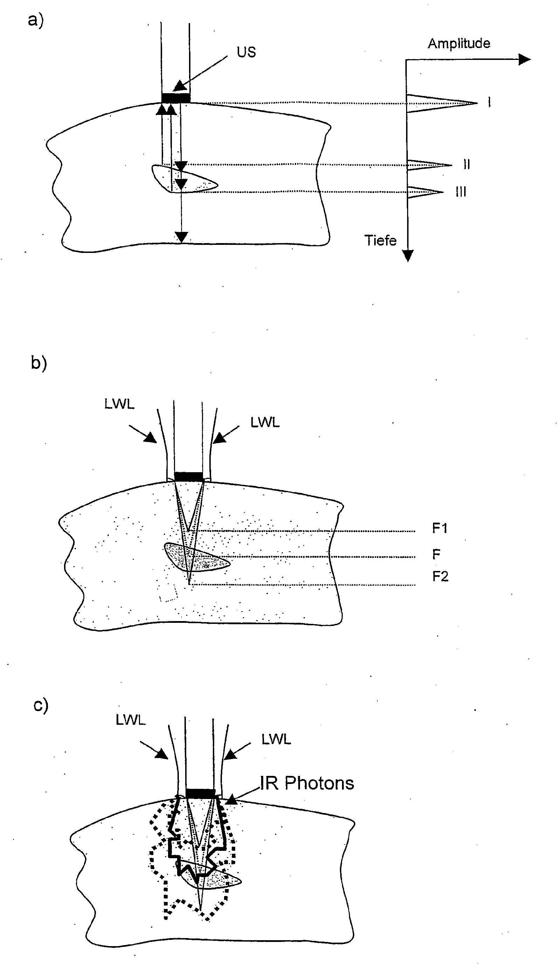

- Fig. 1: schematische Darstellung der in der Vorrichtung implementierten Vorgehensweise zum Auffinden der aussagekräftigsten Fokuslage für die Gewebeklassifizierung

In der bevorzugten Ausführungsform der erfindungsgemäßen Vorrichtung sind - Ultraschall-Transducer-Array, eine Mehrzahl von Lichtquellen und ein lichtempfindliches Sensorarray nebeneinander angeordnet und in einem von Hand haltbaren Applikator integriert. Vorzugsweise sind Lichtquellen und Sensorarray konzentrisch um das Transducer-Array herum angeordnet. Der Applikator sollte auf der Oberfläche des zu untersuchenden Gewebes (Patientenhaut) vorzugsweise befestigt werden, etwa durch Unterdruck oder einen medizinischen Kleber.

Wie in Fig. 1 a) dargestellt, beginnt der Applikator die Untersuchung mit einer Gewebeabtastung, um interessierende Regionen anhand von Impedanzkontrasten zu lokalisieren. Das Transducer-Array (US) strahlt zunächst gefächerten Ultraschall ein, und die Laufzeiten reflektierter Signale werden von der Kontrollvorrichtung ermittelt. Diese Laufzeiten werden in Koordinaten des näher zu untersuchenden, möglicherweise abnormen Gewebes umgerechnet. Aus den Koordinaten werden die Ansteuerparameter der einzelnen Transducer-Elemente in an sich bekannter Weise bestimmt, mit denen das Erzeugen und ggf. Verschieben eines Ultraschallfokus in einem Zielgebiet, das das abnorme Gewebe enthält, möglich ist. Die Koordinaten des Zielgebiets werden ebenso an den Prozessrechner übermittelt, der für das Auslesen des optischen Sensorarrays und das Berechnen der optischen Parameter zuständig ist.

Nach dem Ermitteln des Zielgebiets wird Licht geringer spektraler Breite, bevorzugt Laserlicht, in das Gewebe eingestrahlt, wobei zugleich ein Ultraschallfokus gebildet wird. In Fig. 1 b) wird das Licht über Lichtwellenleiter (LWL) neben die Ultraschallquelle geführt, von wo es ins Gewebe eintritt. Die Lichtquellen selbst müssen also nicht unbedingt in den Applikator integriert werden, sondern nur Mittel zum Führen des Lichts. Fig. 1 b) zeigt ferner, dass zwei Fokuspositionen in den Tiefen F1 und F2 außerhalb des Zielgebiets eingerichtet werden, um dort die optischen Parameter des gesunden Gewebes als Referenz aufzunehmen. Die Aufzeichnung einer Referenz zu Beginn einer Klassifizierungsprozedur ist meistens notwendig und immer empfehlenswert, schon weil sich verschiedene Patienten wesentlich unterscheiden und sogar beim selben Patienten eine zeitliche Abhängigkeit der Messergebnisse bestehen kann (etwa wiederholte Messungen an verschiedenen Tagen).

Die Abweichungen der Messwerte im Zielgebiet von denen des Normalgewebes definieren hier die algorithmisch zu maximierende Funktion. Dazu werden die rückgestreuten Lichtintensitäten vom Sensorarray gemessen, vom Prozessrechner in Anteile aufgeteilt, die den Ultraschallfokus durchlaufen haben oder nicht, und es werden die optischen Parameter der Fokusregion berechnet. Mit den von der Kontrollvorrichtung übermittelten Koordinaten der aktuellen Fokusposition ergibt sich im Prozessrechner eine numerische Funktion, die stützstellenweise abgetastet werden kann. Da hier nur das Maximum der Funktion gesucht wird, kann die Abtastung sprunghaft unter Benutzung bekannter Optimierungsalgorithmen erfolgen. Der Prozessrechner verwendet die optischen Messdaten und besagte Algorithmen unmittelbar dazu, um der Kontrollvorrichtung die Repositionierung des Fokus für die nächste Stützstelle zu befehlen.

Die Iteration der Fokusposition endet automatisch, sobald der Fokus im Gewebe der stärksten Anomalie zu liegen kommt. Es kann vorteilhaft sein, weitere konvergenzerzwingende Kriterien programmtechnisch vorzusehen, etwa im einfachsten Fall der Abbruch der Iteration nach einer festgelegten Zahl von Iterationsschritten.

Im konkreten Beispiel der Fig. 1 werden zwei initiale Messorte in den Tiefen F1 und F2 eingestellt. Die Messwerte können z.B. gemittelt werden und als Referenzwert für das Normalgewebe dienen. Ebenso kann nun ein dritter Messwert bei einer Fokuslage im Zielgebiet (Tiefe F) bestimmt werden, der mit beiden Werten bei F1 und F2 getrennt verglichen wird. Die Wahl und Anzahl der initialen Fokuspositionen hängt unter anderem vom Iterationsalgorithmus ab und soll deshalb hier nicht als einschränkend für die Erfindung verstanden werden. Insbesondere kann es für manche Optimierungsalgorithmen vorteilhaft sein, die Initialstützstellen zufällig auszuwählen.

In Fig. 1 c) sind schematisch einige Streupfade eingestrahlter IR-Photonen zu sehen, die ihren Weg zum Lichtwellenleiter (LWL) zurückfinden, wobei diese jeweils einen Fokus durchlaufen haben. Grundsätzlich können die Photonen wieder in den LWL eintreten und zu einem Detektor geführt werden. Schon aus Gründen der geringen rückgestreuten Intensität ist es aber zu bevorzugen, ein flächiges Sensorarray als Lichtdetektor unmittelbar auf dem zu untersuchenden Gewebe zu platzieren (nicht dargestellt) und die Intensität integrierend über alle Arrayelemente aufzuzeichnen. Das Sensorarray sollte ohnehin eine laterale Ausdehnung aufweisen, die dem Umstand gerecht wird, dass das rückkehrende Licht dazu tendiert, desto weiter seitlich versetzt auszutreten, je tiefer es im Gewebe gestreut wird. Dieser empirisch bekannte Zusammenhang kann im Übrigen auch unterstützend dazu genutzt werden, das im Ultraschallfokus zurück gestreute Licht zu isolieren, da die Tiefe des Fokus ja stets bekannt ist.

Zusammenfassend vollführt die erfindungsgemäße Vorrichtung zwei Aufgaben:
1. Sie nutzt Ultraschall und rückgetreutes IR-Licht zum vollautomatischen Auffinden einer für die Gewebeklassifizierung anhand optischer Parameter maximal aussagekräftigen Position des Ultraschallfokus mittels eines implementierten Optimierungsalgorithmus.
2. Sie untersucht das Gewebe im zuvor optimal positionierten Ultraschallfokus - und nur dort - hinsichtlich seiner optischen Parameter für eine Mehrzahl festgelegter IR-Wellenlängen und nimmt anhand des Vergleichs dieser Messwerte mit tabellierten Befunden pathologischer Untersuchungen eine Klassifizierung des beobachteten Gewebes vor.

Idealerweise, schon wegen der oben erwähnten Abweichungen zwischen in vivo Gewebe und extrahierten Gewebeproben, gibt der Prozessrechner neben der Klassifizierung auch eine Wahrscheinlichkeit der Richtigkeit seiner Analyse aus, um den behandelnden Arzt bei der Entscheidung über weitere Maßnahmen zu unterstützen.

Eine vorteilhafte Ausgestaltung der Erfindung besteht darin, die gemessenen Parameter im Einzelfall abzuspeichern, wenn der Arzt sich für eine Gewebeentnahme und Laboruntersuchung entscheidet. Die Laborergebnisse können dann über eine Schnittstelle, z.B. ein maskengeführtes Eingabeprogramm auf dem Prozessrechner, zusammen mit den gespeicherten Messdaten eingegeben werden, um so den Datenbestand, der zur Klassifizierung herangezogen wird, nach und nach zu erweitern.

## Patentansprüche

1. Verfahren zur in vivo Gewebeklassifizierung lebenden Gewebes, unter Verwendung eines Ultraschallwandler-Arrays, einer Kontrollvorrichtung für das Wandlerarray, wenigstens einer Lichtquelle mit geringer spektraler Breite im Wellenlängenbereich oberhalb von 500 nm, wenigstens eines Lichtdetektors und eines Prozessrechners zur Verarbeitung der Messwerte des Lichtdetektors, wobei der Lichtdetektor nur aus dem Gewebe zurück gestreutes Licht erfasst, das Ultraschallwandler-Array während der Beleuchtung mit dieser wenigstens einer Lichtquelle fokussierten Ultraschall in das Gewebe einstrahlt und der Prozessrechner den Beitrag des im Ultraschallfokus gestreuten Lichts zur gesamten vom Lichtdetektor gemessenen Lichtintensität isoliert und daraus optische Parameter für das Gewebe im Ultraschallfokus berechnet, **dadurch gekennzeichnet, dass**
der Prozessrechner aus den berechneten optischen Parametern die Abweichung dieser optischen Parameter von während der Messung aufgenommenen Referenzwerten in gesundem Gewebe als Kenngröße ableitet, die hinsichtlich eines vorgegebenen Optimalitätskriteriums optimiert wird, indem die Position des Ultraschallfokus im Gewebe durch die Kontrollvorrichtung nach Maßgabe des Prozessrechners verändert wird, und der Prozessrechner die optischen Parameter in der aufgefundenen optimalen Position des Ultraschallfokus mit einer gespeicherten Datentabelle vergleicht und das Gewebe **dadurch** klassifiziert.

2. Verfahren nach Anspruch 1, **dadurch gekennzeichnet, dass** das vorgegebene Optimalitätskriterium die Maximierung der Kenngröße ist.

3. Verfahren nach Anspruch 1 oder 2, **dadurch gekennzeichnet, dass** vor der Messung der optischen Parameter eine Ultraschallabtastung durchgeführt wird, bei der die Konteollvorrichtung die Laufzeiten reflektierter Schallwellen aufzeichnet und daraus eine Region des zu klassifizierenden Gewebes bestimmt wird, in dem Ultraschallfokus zu bilden ist.

4. Verfahren nach einem der Ansprüche 1 bis 3, **dadurch gekennzeichnet, dass** die im Prozessrechner gespeicherte Datentabelle Gewebeklassifizierungen und deren optische Parameter aus ex vivo Messungen umfasst.

5. Verfahren nach Anspruch 4, **dadurch gekennzeichnet, dass** der Prozessrechner gemessene optische Parameter optional speichert und eine Nutzerschnittstelle aufweist, über die den gespeicherten Parametern Gewebeklassifizierungen zuordenbar sind, wobei die gespeicherte Datentabelle fortgeschrieben wird.

6. Verfahren nach einem der Ansprüche 1 bis 5, **dadurch gekennzeichnet, dass** der Prozessrechner beim Vergleich der optischen Parameter mit der gespeicherten Datentabelle eine Wahrscheinlichkeit für die Richtigkeit der Klassifizierung berechnet und ausgibt.

## Claims

1. A method for in vivo tissue classification of living tissue using an ultrasound transducer array, a controller for the transducer array, at least one light source with a narrow spectral range in the wavelength range above 500 nm, at least one light detector and a process computer to process the measurements from the light detector, wherein the light detector captures only light which is scattered back from the tissue, during illumination with said at least one light source, the ultrasound array radiates focussed ultrasound into the tissue and the process computer isolates the contribution of the light scattered in the ultrasound focus to the total light intensity measured by the light detector and computes therefrom optical parameters for the tissue in the ultrasound focus, **characterized in that**
the process computer derives from the computed optical parameters the deviation of said optical parameters from reference values recorded in healthy tissue during the measurement, which is optimized as regards a predetermined optimization criterion, in which the position of the ultrasound focus in the tissue is altered by the controller in response to the process computer, and the process computer compares the optical parameters in the determined optimal position of the ultrasound focus with a stored data table and thereby classifies the tissue.

2. The method according to claim 1, **characterized in that** the predetermined optimization criterion is the maximization of the parameter.

3. The method according to claim 1 or claim 2, **characterized in that** prior to measuring the optical parameter, an ultrasound scan is carried out wherein the controller records the propagation times of reflected sound waves and determines therefrom a region of the tissue to be classified in which the ultrasound focus is to be formed.

4. The method according to one of claims 1 to 3, **characterized in that** the data table stored in the process computer comprises tissue classifications and their optical parameters from ex vivo measurements.

5. The method according to claim 4, **characterized in that** the process computer optionally stores measured optical parameters and has a user interface via which tissue classifications can be assigned to the stored parameters, wherein the stored data table is updated.

6. The method according to one of claims 1 to 5, **characterized in that** when comparing the optical parameters with the stored data table, the process computer computes and disseminates a probability for the accuracy of the classification.

## Revendications

1. Procédé pour la classification de tissus in vivo, employant un système de convertisseurs à ultra-sons, un dispositif de contrôle pour le système de convertisseurs, au moins une source lumineuse avec une largeur spectrale étroite dans la plage de longueurs d'onde supérieure à 500 nm, au moins un photodétecteur et un calculateur de procédé pour traiter les valeurs de mesure du photodétecteur, sachant que le photodétecteur détecte uniquement la lumière diffusée en retour du tissu, le système de convertisseurs irradie dans le tissu les ultra-sons mis au point pendant l'éclairage avec cette au moins une source lumineuse et le calculateur de procédé isole la part de lumière diffusée dans la mise au point d'ultra-sons en l'intensité lumineuse totale mesurée par le photodétecteur et en calcule des paramètres optiques pour le tissu dans la mise au point d'ultra-sons, **caractérisé en ce que**
le calculateur de procédé déduit des paramètres optiques calculés l'écart de ces paramètres optiques avec des valeurs de référence enregistrées durant le mesurage dans le tissu sain en tant que grandeur caractéristique, laquelle est optimisée en regard d'un critère d'optimalité prédéfini, **en ce que** la position de la mise au point d'ultra-sons dans le tissu est modifiée par le dispositif de contrôle d'après la détermination du calculateur de procédé, et le calculateur de procédé compare les paramètres optiques dans la position optimale trouvée de la mise au point d'ultra-sons à un tableau de données enregistré et classifie ainsi le tissu.

2. Procédé selon la revendication 1, **caractérisé en ce que** le critère d'optimalité prédéfini est la maximisation de grandeur caractéristique.

3. Procédé selon la revendication 1 ou 2, **caractérisé en ce qu'**avant le mesurage des paramètres optiques, on réalise un balayage par ultra-sons, au cours duquel le dispositif de contrôle enregistre les durées d'action des ondes sonores réfléchies et une région du tissu à classifier en est déterminée dans laquelle la mise au point d'ultra-sons doit être formée.

4. Procédé selon l'une des revendications 1 à 3, **caractérisé en ce que** le tableau de données enregistré dans le calculateur de procédé comprend des classifications des tissus et leurs paramètres optiques issus de mesures ex vivo.

5. Procédé selon la revendication 4, **caractérisé en ce que** le calculateur de procédé enregistre en option des paramètres optiques mesurés et présente une interface utilisateur par laquelle les classifications des tissus peuvent être attribuées aux paramètres enregistrés, sachant que le tableau de données est actualisé.

6. Procédé selon l'une des revendications 1 à 5, **caractérisé en ce que** le calculateur de procédé, lors de la comparaison des paramètres optiques avec le tableau de données enregistré, calcule et fournit une probabilité d'exactitude de la classification.
